# EUROPEAN PATENT SPECIFICATION

(11) **EP 1 803 423 B1**
(45) Date of publication and mention of the grant of the patent: **18.12.2013**
(21) Application number: 06256629.4
(22) Date of filing: 29.12.2006
(51) Int. Cl.: A61F 2/07

(54) **Stent delivery system with improved delivery force distribution**
System zum Einführen eines Stents mit verbesserter Einführkraftverteilung
Système de pose de stent ayant une meilleure répartition de force

(30) Priority: 30.12.2005 US 322968
(43) Date of publication of application: 04.07.2007
(73) Proprietor: Cordis Corporation, Miami Lakes, FL 33014 (US)
(72) Inventor: Vaughan, Dennis, North Miami, FL 33168 (US)
(74) Representative: Belcher, Simon James

(56) References cited:
- WO-A-00/71059
- WO-A-02/32496
- WO-A-2008/127876
- US-A1- 2005 288 763

## Description

The invention generally relates to a stent delivery system. More specifically, the invention relates to a stent delivery system that improves emplacement of one or more stents or stent grafts by better distributing delivery forces during delivery thereof to an intended treatment site.

Balloon expandable stents and self-expanding stents are generally delivered in a cylindrical form, compressed to a smaller diameter, and placed within a vessel using a catheter-based delivery system. A femoral artery in the groin is typically accessed to deliver the stent graft to its intended position in the abdominal region, for example, the abdominal aortic artery. A guidewire is threaded from the femoral artery access and through the blood vessel to a point beyond the diseased part of the artery.

The delivery system, carrying the stent or stent graft within it, is manipulated through the blood vessel and over the guidewire to a position beyond the diseased area of the blood vessel. Fluoroscopy (x-ray imaging) is typically used to help guide the delivery system and the stent or stent graft to the intended treatment site. Once the stent or stent graft is located as desired, an outer sheath of the delivery system is gradually withdrawn to enable the stent or stent graft to expand and anchors itself to the inside of the vessel. Some stent or stent grafts require inflation of a balloon in order to expand within the vessel. Other stent grafts are comprised of self-expanding materials that expand upon withdrawal thereof from within the delivery system sheath. Hooks or barbs at ends, or other positions, of the stent may be used to help anchor the stent to the vessel walls.

Typically, delivery of the stent or stent graft requires a push rod, or other member, that engages a proximal end of the stent or stent graft during deployment thereof from the delivery catheter. Although engaging the proximal end of the stent or stent graft as the sheath is withdrawn helps to maintain the proximal end of the stent or stent graft in place, such engagement may also cause more distal segments of the stent or stent graft, or adjacent stents or stent grafts, to buckle with one another.

To overcome the above cited buckling of adjacent segments, stents or stent grafts, some delivery systems incorporate a release wire that anchors to a distal most segment of a stent or stent graft. The release wire thus maintains the segments, stent or stent graft in the appropriate alignment relative to one another even as the outer sheath is withdrawn or the push rod is engaged against a proximal end of the stent or stent graft. After deployment, the release wire is independently withdrawn through the catheter. Although the release wire tends to provide good placement of a stent or stent graft, such a release wire configuration increases the steps necessary for delivering a stent or stent graft to an intended treatment site and may limit the reduction of delivery catheter French size due to inclusion of such a release wire and components associated therewith. Moreover, a release wire delivery system tends to be costly and more complicated to manufacture,

WO-00/71059A1 relates to stent delivery system for preventing kinking during introduction of the delivery system through tortuous anatomy. It comprises docking sections at a catheter tip and at a pusher proximal end to hold a compressed stent. The docking section may be radially-biased-outward.

In view of the above, a need exists for systems and methods that simplifies delivery of a stent or stent graft to an intended treatment site, and that better distributes delivery forces throughout a stent or stent graft during delivery thereof, while minimizing the costs and manufacturing complexity of the delivery system.

The invention comprises a stent or stent graft delivery system as defined in appended claim 1 for use with one or more expandable stents or stent grafts. The one or more expandable stents or stent grafts may comprise a series of adjacent segments having voids therebetween such segments of a single stent or stent graft, or may comprise one or more adjacent stents or stent grafts having voids between such adjacent stents or stent grafts. In any case, the delivery system comprises a catheter having a lumen therethrough, an inner core over which the one or more stents or stent grafts is mounted for delivery from the catheter, and an outer sheath overlying the one or more stent or stent graft and the inner core. Whereas conventional stent delivery systems comprise an independent push rod that engages a proximal end of the stent to be delivered, the inner core of the delivery system described herein further comprises a series of members that flare outwardly from the inner core and towards the outer sheath to engage segments or fill voids in or between the stent, stents or stent grafts, respectively. The outwardly flared members of the inner core thus help maintain the segments of the stent or the one or more stents or stent grafts in spaced relation with respect to one another as delivery thereof through and catheter and removal of the outer sheath occurs to position the one or more stents or stent grafts at the intended treatment site. The outwardly flared members thus help to distribute delivery forces more evenly throughout the one or more stents or stent grafts being delivered from the catheter. The flared segmented members are axially slit to comprise collapsible fingers, the ends of which fill or engage the respective segments, voids and portions of the one or more stents or stent grafts during delivery thereof.

In some embodiments, the outwardly flared members are integrally molded with the inner core and increasingly taper proximally to distally such that the smaller diametered proximal portion of each flared member eventually blends into the inner core, whereas the larger diametered distal portion of each flared member extend to engage segments of the single stent or to fill or engage respective voids and engage portions of the one or more stents or stent grafts during delivery thereof. Ideally, the larger diametered distal portion of the outwardly flared members engage a proximal portion of each adjacent stent or stent graft to maximize the distribution of delivery forces thereto the one or more stents or stent grafts. A biocompatible, lubricious material preferably comprises the inner core and the outwardly flared members. Each successive flared member of the series of flared members is similarly configured. This configuration provides an inner member with sufficient columnar strength to maintain the one or more stents or stent grafts in spaced relation to one another during delivery thereof, and to effect delivery of the one or more stents or stent grafts through the catheter to the intended treatment site without the need for more complex release wires or other instruments or steps. This configurations also enables easy removal of the inner core after deployment of the one or more stents or stent grafts has occurred at the intended treatment site as a result of the lesser diametered proximal portion of each flared member leading the withdrawal of the inner core from the patient after the one or more stents or stent grafts has resumed its expanded state and disengaged the larger diametered flared member portions from the one or more stent or stent graft.

The present invention can be used in a method for delivering one or more stents or stent grafts via a catheter-based delivery system, the method comprising:
mounting one or more expandable stents or stent graft in a crimped state to an inner core of the catheter-based delivery system;
engaging outwardly flared members of the inner core to portions of the one or more stents or stent grafts to maintain the one or more stents in a desired spatial orientation during delivery thereof to an intended treatment site;
disposing an outer sheath over the one or more stents or stent grafts;
inserting the delivery system to the intended treatment site;
distributing delivery forces throughout the one or more stents or stent grafts during delivery thereof by the engagement of the outwardly flared members with the portions of the one or more stents or stent grafts; and
withdrawing the outer sheath and resuming an expanded state of the one or more stents thereby disengaging the outwardly flared members from the one or more stents or stent grafts; and
withdrawing the inner core.

The method may further comprise axially slitting portions of the outwardly flared members from a distal end thereof towards the inner core of the catheter-based delivery system.

The method may also further comprise forming collapsible fingers from the outwardly flared members by the axially slitting thereof. Engaging the outwardly flared members may further comprise engaging some of the collapsible fingers with the portions of the one or more stent or stent grafts by penetration of the some of the collapsible fingers through voids in the one or more stents or stent grafts, while confining others of the collapsible fingers from such engagement due to solid portions of the one or more stent or stent grafts. The method may further comprise flexing the collapsible fingers into recesses in the inner core to minimize a delivery profile of the one or more stents or stent grafts.

Embodiments of the invention will now be described by way of example with reference to the accompanying drawings, in which:
Figure 1 illustrates an exemplary conventional catheter-based stent delivery system.
Figure 2 illustrates an embodiment of a catheter-based stent delivery system according to the invention.
Figure 3 illustrates a single stent mounted onto an inner core of the delivery system of Fig. 2 according to the invention.
Figures 4 illustrates multiple stents mounted onto an inner core of the delivery system of Fig. 2 according to the invention.
Figure 5 illustrates a stent graft mounted onto an inner core of the delivery system of Fig. 2 according to the invention.
Figures 6A & 6B illustrates another embodiment of the inner core according to the invention.

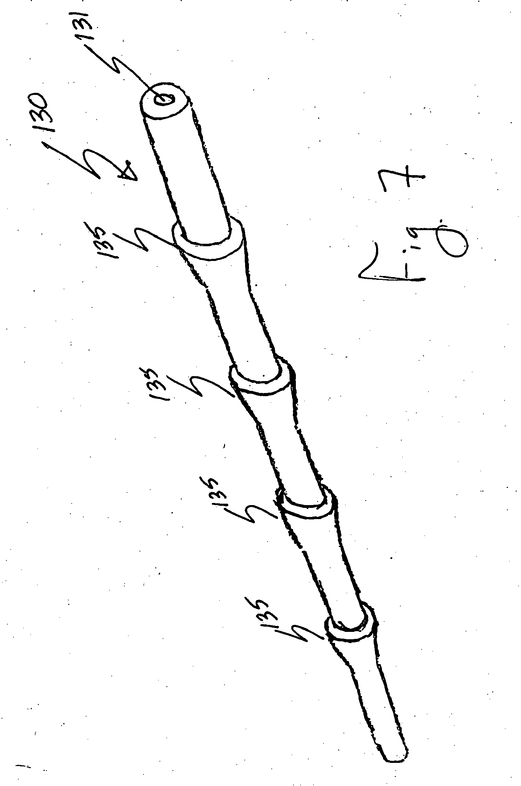

Figure 7 illustrates an example of outwardly flared members integrally molded with the inner core.

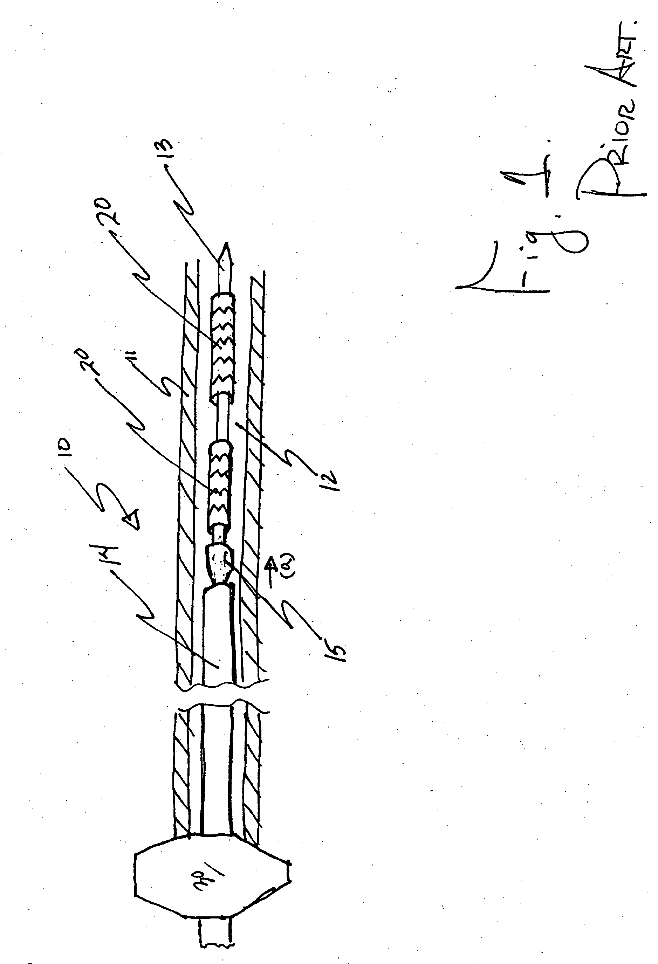

Fig. 1 illustrates a conventional catheter-based stent delivery system 10 having one or more stents 20 delivered therefrom to an intended treatment site. As shown in Fig. 1, the conventional stent delivery system 10 comprises a catheter 11 having a lumen 12 extending therethrough, an inner member 13, an outer sheath 14, and a pushrod 15. A hub 30 may be provided to help the operator handle insertion and withdrawal thereof. The pushrod 15 is moved in the direction of arrow (a) in order to maintain the one or more stents 20 in place as the outer sheath 14 is withdrawn to deploy the stents 20 as desired, or the pushrod is moved in the direction of arrow (a) in order to help push the one or more stents 20 into the desired deployed position. In either case, the use of the pushrod 14 against the proximal end of the one or more stents 20 tends to buckle the stents 20 or otherwise alter the spacing within or between the one or more stents 20. The altered spacing often results in an undesirable positioning of the one or more stents 20 relative to the intended treatment site. Moreover, where buckling within the one or more stents 20 occurs, such buckling risks injury to the vasculature of the patient and typically requires redeployment of the one or more stents 20. Buckling often also requires the use of new stents to replace the damaged or buckled stents 20 so as to reduce the risk of injury to the patient and to increase the efficacy of the one or more stents at the intended treatment site.

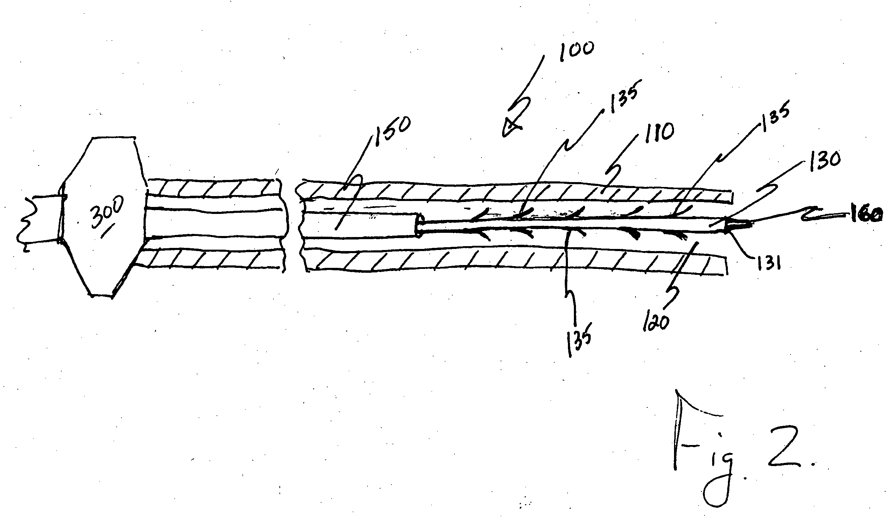

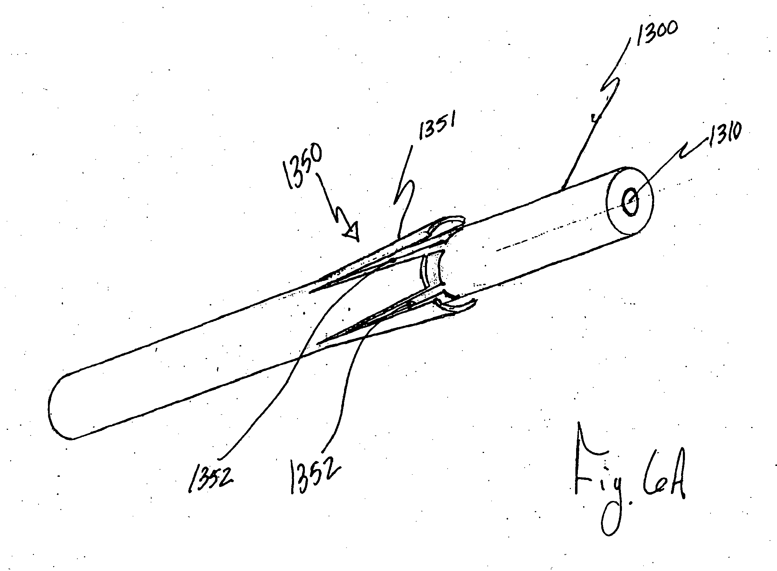

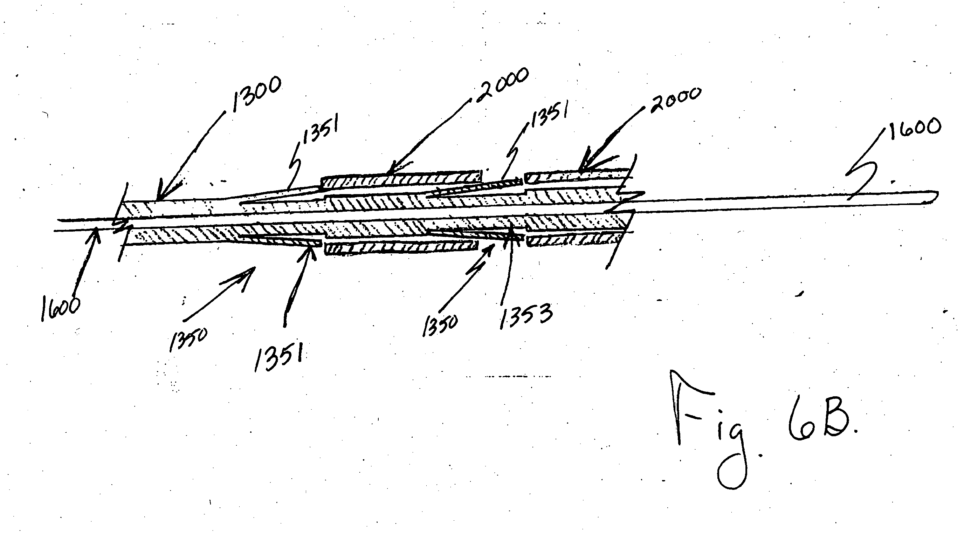

Fig. 2 illustrates an embodiment of a catheter-based stent delivery system 100 according to the invention described herein. As shown in Fig. 2, the stent delivery system 100 comprises a catheter 110 with a lumen 120 extending therethrough, an inner core 130,

and an outer sheath 150. The inner core 130 further comprises a central lumen 131 extending through the inner core 130 and through which lumen 131 a guidewire 160 is provided to help locate the catheter 110 as desired. The inner core 130 further comprises a series of outwardly flared members 135 extending outwardly from the inner core 130. The outwardly flared members 1350 are integral with the inner core 1300 as a series of collapsible fingers 1351, as in Figs. 6A & 6B, for example.

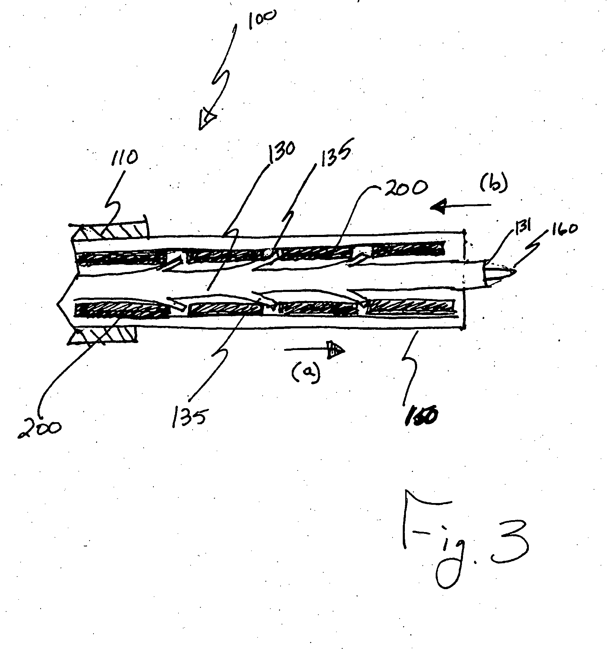

Referring again to Fig. 2, a hub 300 may be provided to help insertion and withdrawal of the system 100 by the operator. One or more stents or stent grafts 200 are mounted onto the inner core 130 (Fig. 3) for delivery therefrom the stent delivery system 100. Each outwardly flared member 135 of the series of outwardly flared members 135 comprises a larger diametered distal portion and a smaller diametered proximal portion, wherein distal is understood herein to refer to locations furthest from the medical operator and proximal is understood to refer to locations closest to the medical operator. The smaller diametered proximal portion of each flared member 135 generally blends into the inner core 130, and the larger diametered distal portion of each flared member 135 engages a segment or a portion of the one or more stents or stent grafts mounted on the inner core 130 for eventual delivery therefrom.

Fig. 3 illustrates a single stent 200 mounted, in its crimped state, onto an inner core 130 of the stent delivery system 100 of Fig. 2, for example. The larger diametered distal portion of the outwardly flared members 135 of the inner core 130 project through voids in the stent 200 to engage axially adjacent segments of the stent 200. The outwardly flared member 135 includes axial slits so as to comprise collapsible fingers 1351 from the outwardly flared member as in Figs. 6A & 6B, for example, wherein the outwardly flared members are identified as 1350. Referring still to Figs. 6A & 6B, the outwardly flared member 1350 having collapsible fingers 1351 permit some of the collapsible fingers 1351 to project through voids in the stent 2000 to engage segments of the stent 2000 during, while others of the collapsible fingers 1351 are confined from engaging the stent 2000 in the same manner by solid portions of the stent. The engagement of the axially adjacent segments of the stent 200, or 2000, in this manner by the flared members 135, or collapsible fingers 1351 of flared members 1350, help to maintain the spatial orientation of the stent and segments thereof as delivery of the stent is effected.

In particular, referring again to Fig. 3, after mounting of the stent 200 onto the inner core 130 as described above, delivery of the stent 200 is effected by movement of the inner core 130 in the direction of arrow (a), whereafter the outer sheath 150 is withdrawn in the direction of arrow (b) when the stent 200 has reached the intended treatment site. Upon withdrawal of the outer sheath 140 by movement in the direction of arrow (b), the expandable stent 200 resumes its expanded state within the blood vessel, thereby disengaging the stent 200 from the larger diametered portions of the respective flared members 135. The inner core 130 is thereafter withdrawn by movement thereof in the direction of arrow (b), while the stent 200 remains deployed within the blood vessel at the intended treatment site.

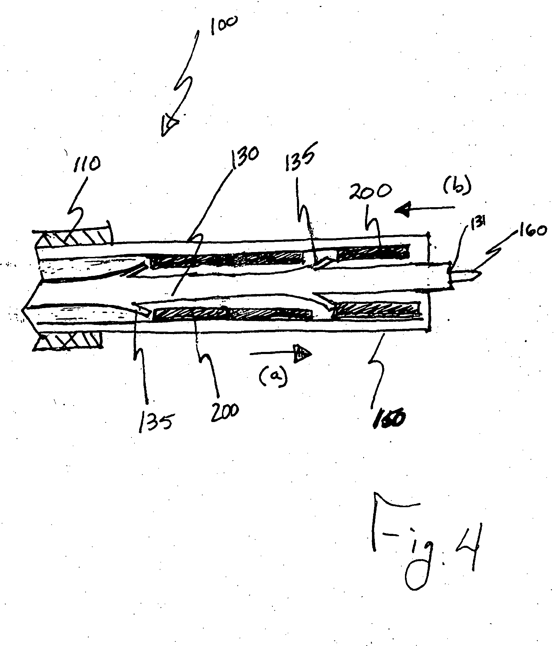

Although the mounting and delivery of a single stent 200 is described above, the artisan will readily appreciate that a series of one or more stents or stent grafts may be instead, or additionally, be mounted onto the inner core 130 for delivery from the stent delivery system 100 otherwise described above. The multiple stents 200 may be the same size and length, or may be different sizes or lengths, in order to accommodate different physiological needs in the discretion of the medical practitioner. Spacing between the outwardly flared members 135 on the inner core 130 may be varied to accommodate the different sizes or lengths of multiple stents, or the inner core 130 with one spacing arrangement between the outwardly flared members 135 may be interchangeably used with variously lengthed or sized stents. In any event, where multiple stents 200 are mounted for delivery from the stent delivery system 100, the outwardly flared members 135 of the inner core ideally engage proximal ends of the stents 200, although engagement of other portions of the stents 200 through voids therein, may also be used. Engagement of the multiple stents 200 in this manner helps to distribute delivery forces more equally among the stents 200, while maintaining the spatial orientation of the stents 200 as delivery thereof is effected. Fig. 4 illustrates an example of multiple stents 200 mounted, in their crimped state, on an inner core 130 as described above. Delivery thereof the multiple stents 200 is otherwise effected as described above with respect to a single stent 200, as the artisan will readily appreciate.

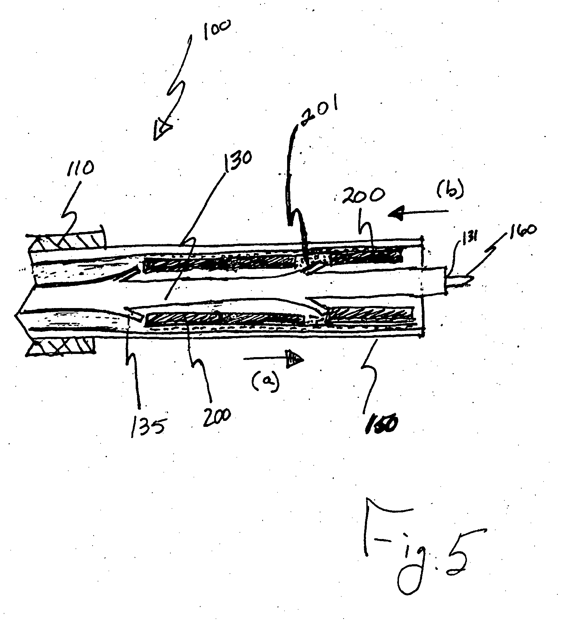

Fig. 5 illustrates the case where the stent is a stent graft 200 having graft material 201 associated therewith. Where one or more stent grafts 200 is deployed via the stent delivery system 100 described herein, the stent grafts 200 are mounted to the inner core 130 as in earlier described embodiments. When crimped onto the inner core 130, the outwardly flared members 135 displace the graft material 201 otherwise existing in the voids of the stent graft whereat the outwardly flared members 135 engage portions of the stent graft 200 for delivery from the catheter-based delivery system 100. Ideally, as before, the outwardly flared members 135 engage the proximal ends of the one or more stent grafts 200 in order to help maintain the spatial orientation of the stent graft 200 and to help distribute delivery forces more evenly throughout the stent graft 200 as the stent graft is delivered to the intended treatment site. In practice, the delivery of the one or more stent grafts 200 using the catheter-based delivery system having the inner core 130 and series of outwardly flared members 135 described herein is as described above, as the artisan will readily appreciate. Thus, a single stent graft, or multiple stent grafts, may be delivered similar to the delivery of a single stent, or multiple stents, as otherwise described above.

Preferably, the inner core 130 is a solid tubular piece having the outwardly flared members 135 made integrally therewith. The inner core 130 and outwardly flared members 135 are comprised of a biocompatible, lubricious material, such as Pebax, for example. Of course, the artisan will appreciate that other known or later developed biocompatible, lubricious materials may also comprise the inner core 130 and outwardly flared members 135 instead of, or in addition to, the Pebax, for example. Such other materials may comprise biocompatible plastics, polymers, or blends thereof, for example. Because imbedment of the inner core 130 or outwardly flared members 135 into the one or more stents or stent grafts, or segments thereof, is not required according to the delivery system and techniques described herein, a compressible material is not required to comprise the inner core or outwardly flared members.

Although described above in the context of a solid inner core 130, Fig. 6A & 6Billustrates aspects of an alternative embodiment of an inner core 1300 usable with the delivery system otherwise described herein. In particular, Fig. 6A illustrates the inner core 1300 having outwardly flared members 1350 along portions thereof. Although only one outwardly flared member is shown in Fig. 6A, the artisan will readily appreciate that an inner core 1300 may also be comprised of a series of similarly configured outwardly flared members 1350. Fig. 6B, for example, shows in cross-section, an inner core 1300 comprised of a series of outwardly flared members 1350 having collapsible fingers 1351 engaging portions of one or more stents or stent grafts 2000. The outwardly flared members 1350 further comprise collapsible fingers 1351 formed by axial slits 1352 provided in the outwardly flared members. The axial slits 1352 generally extend from a distal tip of the outwardly flared member to the outer surface of the inner core 1300. In this manner, the collapsible fingers 1351 are able to accommodate engagement of a variety of stent or stent graft designs, whereby some of the collapsible fingers 1351 will project through voids in the stent or stent graft 2000 to engage segments thereof for delivery, whereas others of the collapsible fingers 1351 will be confined from engaging segments of the stent or stent graft 2000 in the same manner by solid portions of the stent or stent graft. The collapsible fingers 1351 thus flex inwardly away from the one or more stents or stent grafts 2000 mounted thereon the inner core 1300 to reduce delivery forces experienced during delivery, which generally occurs as otherwise described above. The flexible nature of the collapsible fingers 1351 helps the inner core 1300 more readily conform to irregularities exhibited in the interior diameter of the one or more stents or stent grafts during delivery, or during crimping thereof onto the inner core 1300. As shown in Figs. 6A & 6B, the collapsible fingers 1351 also flex inwardly into recesses 1353 formed in the inner core 1300 during delivery of the one or more stents or stent grafts 2000, which also helps minimize the profile thereof during delivery.

The material described herein is not limited to the materials, designs or shapes referenced herein for illustrative purposes only, and may comprise various other materials, designs or shapes suitable for the systems and methods described herein, as should be appreciated by the artisan.

## Claims

1. A stent delivery system for use with expandable stents, the stent delivery system comprising:
a catheter (110) having a lumen (120) extending therethrough;
an inner core (130, 1300) having a single stent or stent graft (200, 2000), or one or more adjacent stents or stent grafts crimped thereon for delivery therefrom; and
an outer sheath (150) overlying the single stent or stent graft (200, 2000), or one or more stents or stent grafts, and the inner core (130, 1300); **characterised in that**
the inner core (130, 1300) comprises a series of outwardly flared members (135, 1350) comprising axial slits (1352) and collapsible fingers (1351) formed thereby wherein the collapsible fingers (1351) extend from the inner core (130, 1300) and flare outwardly from the inner core (130, 1300) and towards the outer sheath (150) engaging or filling voids in or between segments of the single stent or stent graft (200, 2000), or portions of the one or more adjacent stents or stent grafts during delivery thereof; and
the inner core (1300) further comprises recessed areas into which the collapsible fingers (1351) may flex to lower the delivery profile of the one or more stents or stent grafts;
wherein engagement of the segments, adjacent stents, or adjacent stent grafts by the series of outwardly flared members distributes delivery force more uniformly and maintains spacing of the segments, stents or stent grafts during delivery thereof to an intended treatment site.

2. The stent delivery system of claim 1, wherein the outwardly flared members (135, 1350) are integrally molded with the inner core.

3. The stent delivery system of claim 1, wherein each outwardly flared member (135, 1350) increasingly tapers proximally to distally such that each further comprises a larger diametered distal portion and a smaller diametered proximal portion, the smaller diametered proximal portion generally blending into the inner core (130, 1300).

4. The stent delivery system of claim 1, wherein the axial slits (1352) extend from a distal end of the outwardly flared member (1350) towards, but not beyond, the inner core (1300).

## Patentansprüche

1. Stenteinführungssystem zur Benutzung mit expandierbaren Stents, wobei das Stenteinführungssystem Folgendes umfasst:
einen Katheter (110) mit einem Lumen (120), das sich durch diesen erstreckt;
einen inneren Kern (130, 1300) mit einem einzelnen Stent oder Stentgraft (200, 2000) oder einem oder mehreren angrenzenden Stents oder Stentgrafts, die darauf festgeklemmt sind, um diese damit einzuführen; und
eine äußere Ummantelung (150), welche über dem einzelnen Stent oder dem Stentgraft (200, 2000) oder dem einen oder den mehreren Stents oder Stentgrafts und dem inneren Kern (130, 1300) liegt, **dadurch gekennzeichnet, dass** der innere Kern (130, 1300) eine Abfolge von nach außen aufgeweiteten Teilen (135, 1350) umfasst, welche axiale Schlitze (1352) und einfaltbare Finger (1351) umfassen, die mit diesem ausgebildet sind, wobei sich die einfaltbaren Finger (1351) von dem inneren Kern (130, 1300) erstrecken und sich von dem inneren Kern (130, 1300) in Richtung zu der äußeren Ummantelung (150) aufweiten und Leerräume in oder zwischen Segmenten des einzelnen Stents oder des Stentgrafts (200, 2000) oder Abschnitte von dem einen oder den mehreren angrenzenden Stents oder Stentgrafts beim Einführen von diesen ergreifen oder diese ausfüllen; und
der innere Kern (1300) ferner vertiefte Bereiche umfasst, in welche die einfaltbaren Finger (1351) sich biegen können, um das Einführungsprofil des einen oder der mehreren Stents oder Stentgrafts zu verringern;
wobei ein Eingreifen von Segmenten, angrenzenden Stents oder angrenzenden Stentgrafts durch die Abfolge von nach außen aufgeweiteten Teilen eine Einführungskraft gleichmäßiger verteilt und Abstände der Segmente, Stents oder Stentgrafts während dem Einführen von diesen in einen bestimmten Behandlungsort erhält.

2. Stenteinführungssystem nach Anspruch 1, wobei die nach außen aufgeweiteten Teile (135, 1350) integral mit dem inneren Kern geformt sind.

3. Stenteinführungssystem nach Anspruch 1, wobei jedes nach außen aufgeweitete Teil (135, 1350) von der proximalen zur distalen Seite zunehmend konusförmig zuläuft, so dass jedes Weitere einen größeren distalen Durchmesserabschnitt und einen kleineren proximalen Durchmesserabschnitt umfasst, wobei der kleinere proximale Durchmesserabschnitt sich allgemein in den inneren Kern (130, 1300) einfügt.

4. Stenteinführungssystem nach Anspruch 1, wobei sich die axialen Schlitze (1352) von einem distalen Ende eines nach außen aufgeweiteten Teils (1350) in Richtung zum inneren Kern (1300) erstreckt, jedoch nicht darüber hinaus.

## Revendications

1. Système de pose de stent pour utilisation avec des stents aptes à s'expanser, le système de pose de stent comprenant :
un cathéter (110) ayant une lumière (120) s'étendant à travers celui-ci ;
un noyau interne (130, 1300) ayant un stent unique ou une endoprothèse (200, 2000), ou un ou plusieurs stents adjacents ou endoprothèses serties sur celui-ci pour la délivrance de celui-ci ; et
une gaine externe (150) reposant sur le stent unique ou l'endoprothèse (200, 2000) ou un ou plusieurs stents ou endoprothèses, et le noyau interne (130, 1300) ; **caractérisé en ce que**
le noyau interne (130, 1300) comprend une série d'éléments évasés vers l'extérieur (135, 1350) comprenant des fentes axiales (1352) et des doigts aptes à s'affaisser (1351) formés par eux, où les doigts aptes à s'affaisser (1351) s'étendent du noyau interne (130, 1300) et s'évasent vers l'extérieur depuis le noyau interne (130, 1300) et vers la gaine externe (150) en venant en prise avec ou en remplissant des vides dans ou entre des segments du stent unique ou de l'endoprothèse (200, 2000), ou des portions d'un ou de plusieurs stents adjacents ou d'endoprothèses durant la délivrance de ceux-ci ; et
le noyau interne (1300) comprend en outre des zones évidées dans lesquelles les doigts aptes à s'affaisser (1351) peuvent fléchir pour diminuer le profil de délivrance d'un ou de plusieurs stents ou endoprothèses ;
où la mise en prise des segments, stents adjacents ou endoprothèses adjacentes par la série d'éléments évasés vers l'extérieur distribue la force de délivrance plus uniformément et maintient l'espacement des segments, stents ou endoprothèses durant la délivrance de ceux-ci à un site de traitement prévu.

2. Système de pose de stent selon la revendication 1, dans lequel les éléments évasés vers l'extérieur (135, 1350) sont intégralement moulés avec le noyau interne.

3. Système de pose de stent selon la revendication 1, dans lequel chaque élément évasé vers l'extérieur (135, 1350) diminue de plus en plus proximalement à distalement de telle sorte que chaque suivant comprend une portion distale d'un plus grand diamètre et une portion proximale d'un plus petit diamètre, la portion proximale d'un plus petit diamètre se mélangeant généralement avec le noyau interne (130, 1300).

4. Système de pose de stent selon la revendication 1, dans lequel les fentes axiales (1352) s'étendent d'une extrémité distale de l'élément évasé vers l'extérieur (1350) vers, mais non pas au-delà, le noyau interne (1300).
